# EUROPEAN PATENT APPLICATION

(11) **EP 3 188 058 A1**
(43) Date of publication of application: **05.07.2017**
(21) Application number: 15461590.0
(22) Date of filing: 30.12.2015
(51) Int. Cl.: G06F 19/00

(54) **A METHOD AND A SYSTEM FOR ANONYMIZING IMAGE DATA IN MEDICAL IMAGES**

(71) Applicant: Szczepanik, Adam, 93-558 Lodz (PL); Szeler, Tomasz, 93-558 Lodz (PL); Musialek, Jakub, 93-558 Lodz (PL)
(72) Inventor: Musialek, Jakub, 95-050 Konstantynow Lodzki (PL); Podyma, Marek, 93-403 Lodz (PL); Jopek, Lukasz, 92-413 Lodz (PL); Szczepanik, Adam, 90-009 Lodz (PL)
(74) Representative: Kancelaria Eupatent.pl Sp. z.o.o

(57) **Abstract**

A method for anonymizing image data in medical images, characterized in that it comprises the following steps: retrieving (101) the image data from the medical image; processing (102) the image data; analyzing (103) the 2D and 3D image data to automatically detect regions to be anonymized; generating (108) anonymized data on the basis of the regions to be anonymized; and storing (110) the anonymized 2D images, by storing changed image data in a file compliant with a selected standard.

## Description

### TECHNICAL FIELD

The present invention relates to a method and a device for anonymizing image data in medical images, in particular in images of a facial skeleton or a head.

### BACKGROUND

Anonymization of medical data becomes an important issue, in particular due to common storage and management of examination results in a form of a digital imaging. The aim of the anonymization of the medical data is to protect privacy of patients. The medical data, stored in a digital form may be stored on various media types, yet may be also distributed through a computer network (including the Internet).

Popular standards of medical data storage are DICOMS and PACS. These standards allow searching and downloading results of different kinds of examinations, to remote machines, located in a different place than a data repository. Usually, employees of the medical faculty, in which the examination was performed, have access to the medical images. These images may be also shared to other persons, outside that medical faculty, for example to scientists for research purposes. As all data available in a network, the medical images are prone to illegal access by unauthorized entities. Therefore, the number of entities having access to the medical images may be substantial. In order to protect the privacy of a patient, it would be advantageous to anonymize the stored data.

Data of the medical images may be divided into two groups: metadata and image data. In case of the DICOM standard, a significant amount of the metadata is stored in tags, for example defining: information of a type (type of the examination), a method of image compression, a size of the image, and its other technical parameters often essential for a proper display of the examination. Apart from the technical information, the DICOM tags contain also patient information (i.e. name, surname, age, sex, evidence number), information about a person performing the examination, a hospital unit etc.

Various methods for anonymization are known from the following patent publications: US20040078238, EP2815372, WO2013123085, US8553965, US8875034.

The US patent US8553965 discloses a method for anonymizing image data which is based on encrypting all image data. However, encrypting all image data causes the anonymization of not only a patient image, but also the data which is essential from the diagnostic point of view.

It would be advantageous to develop a method for anonymizing image data in medical images, in particular the images of CT and MRI examinations, in order to prevent identification of a patient (in particular, to prevent recognition of facial features and the patient's face), but to keep data that are important from the diagnostic point of view.

### SUMMARY

The object of the invention is a method for anonymizing image data in medical images, characterized in that it comprises the following steps: retrieving the image data from the medical image; processing the image data; analyzing the 2D and 3D image data to automatically detect regions to be anonymized; generating anonymized data on the basis of the regions to be anonymized; and storing the anonymized 2D images, by storing changed image data in a file compliant with a selected standard.

The method may further comprise retrieving metadata characterizing the image data, such as an image size, an origin of a coordinate system or a modality of an examination.

Processing the image data may comprisee segmenting the image to generate an image of tissues.

The segmentation can be performed as a two-stage process, wherein the first stage of the segmentation comprises separating a bone tissue from the image data and wherein the second stage of the segmentation comprises separating classes associated with a soft tissue and separating a skin tissue from a brain tissue and from the other tissues within the patient's body.

The automatic detection of the regions to be anonymized may comprise determining a main anonymization region and characteristic regions of a face.

The method may further comprise after analyzing the 2D and 3D image data, the following steps: presenting the regions to be anonymized, on the basis of the analysis of the 2D and 3D image data; receiving a modification of sizes and shapes of the main anonymization region and determined characteristic features of the face; and/or receiving additional regions or deleting existing detected regions; and/or modifying a range and a degree of modification of values of pixels of the images.

The method may comprise anonymizing the regions covering a head, a fragment of the head, a face or a fragment of the face.

The method may comprise receiving information on whether the anonymization process is to be reversible or irreversible.

Generating the anonymization data may comprise creating a list of all pixels to be anonymized, and next creating minimal parallelepipeds around all the anonymization regions, wherein each anonymization region is processed separately.

The method may comprise subjecting the pixels of the medical image to a modification of a position and/or a value, to prevent identification of characteristic anatomical features of a patient while preserving the diagnostic value of the examination.

The method may comprise enabling the process of a de-anonymization, to allow recovering an initial state of the image data prior to the anonymization, wherein the data can be de-anonymized by subjecting it to the reversible anonymization based on data identifying the type of an encryption algorithm and an encryption key.

The invention also relates to a computer program comprising program code means for performing all the steps of the computer-implemented method as described above when said program is run on a computer, as well as a computer readable medium storing computer-executable instructions performing all the steps of the computer-implemented method as described above when executed on a computer.

There is also disclosed a system for anonymizing image data in medical images, characterized in that it comprises: a server comprising an image segmentation module configured to perform processing the image data and analyzing the 2D and 3D image data to automatically detect regions to be anonymized on the basis of the image data retrieved from an external source; an anonymization module configured to perform generating anonymized data on the basis of the regions to be anonymized and to share a rendered image via the server to an external client; wherein the client is configured to send data, needed for a de-anonymization of the image, and the anonymized images to the server; a de-anonymization module configured to receive the data needed for the de-anonymization of the image and to receive the anonymized images and configured to de-anonymize in case when the image data was subjected to the reversible anonymization and when the module receives the correct data comprising the type of an encryption algorithm and an encryption key; and a memory module for storing the de-anonymized images.

### BRIEF DESCRIPTION OF DRAWINGS

The present invention is presented by means of example embodiments in a drawing, in which:
Fig. 1 shows a flowchart of a method for anonymizing image data in medical images;
Fig. 2 shows a flowchart of a segmentation process;
Fig. 3 shows a flowchart of a de-anonymization of the image data;
Fig. 4 shows a schematic of a system;
Figs. 5A-5D show examples of image data.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

### DETAILED DESCRIPTION

A CT scan of a head of a patient, in particular performed by a high-quality multi-row detector, allows reconstructing many anatomical details of a patient's body, in particular such elements as skin tissue of a face. After a 3D volumetric reconstruction on a display of a monitor, it is possible to obtain a reconstructed face of the patient, which is sufficient to allow recognition of the patient.

However, if the data representing characteristic fragments of a human face (such as eyes, a nose, lips etc.) were erased from the image data, without distorting data responsible for regions essential from a diagnostic point of view, then it would be possible to achieve an image protected from a patient's recognition (i.e. an anonymized image), but still at least partially or even fully valuable from the diagnostic point of view.

Methods of recognition of details of a human face on 3D images are known in the art and are not considered as an essential feature of the presented solution. In the presented solution, various known techniques may be implemented, for example as described in the following documents:
- A. Lanitis, C. J. Taylor, and T. F. Cootes, "Automatic face identification system using flexible appearance models," Image Vis. Comput., vol. 13, no. 5, pp. 393-401, 1995. - use of a model based on analysis of shape and brightness of pixels;
- T. Heimann and H.-P. Meinzer, "Statistical shape models for 3D medical image segmentation: a review," Med. Image Anal., vol. 13, no. 4, pp. 543-563, Aug. 2009 - use of statistical shape models (SSMs), allows identifying various anatomical elements;
- K. H. Höhne and W. A. Hanson, "Interactive 3D segmentation of MRI and CT volumes using morphological operations," J. Comput. Assist. Tomogr., vol. 16, no. 2, pp. 285-294, Mar. 1992 - use of morphological operations;
- M. Bomans, H. K.-H., U. Tiede, and M. Riemer, "3-D segmentation of MR images of the head for 3-D display," IEEE Trans. Med. Imaging, vol. 9, no. 2, pp. 177-183, 1990 - detection and better presentation of soft tissue surface in MRI images with the use of morphological operators and convolution filters;
- T. Weglisnki and A. Fabijanska, "Image segmentation algorithms for diagnostic support of hydrocephalus of children," Automatyka, vol. 15, no. 3, pp. 309-319, 2011 - isolation of bone structures and brain tissue on the basis of 2D cross-sections of a CT examination, with the use of a region-growing method;
- Chia-Chi Teng, T. Chia-Chi, L. G. Shapiro, and I. Kalet, "Automatic Segmentation of Neck CT Images," in 19th IEEE Symposium on Computer-Based Medical Systems (CBMS'06), 2006 - segmentation of a neck region with the use of dynamic thresholding;
- M. Kobashi, K. Masaharu, and L. G. Shapiro, "Knowledge-based organ identification from CT images," Pattern Recognit., vol. 28, no. 4, pp. 475-491, 1995 - shape analysis and segmentation with the use of an active contour method, allowing differentiation of anatomic bone structures from other structures such as blood vessels;
- A. Parraga, P. Adriane, S. Altamiro, P. Johanna, M. Benoit, and D. Mathieu, "Quality Assessment of Non-Rigid Registration Methods for Atlas-Based Segmentation in Head-Neck Radiotherapy," in 2007 IEEE International Conference on Acoustics, Speech and Signal Processing - ICASSP '07, 2007 - segmentation by assigning an existing model to actual data by use of atlas methods and image registration algorithms;
- D. J. Withey and Z. J. Koles, "Medical Image Segmentation: Methods and Software," in 2007 Joint Meeting of the 6th International Symposium on Noninvasive Functional Source Imaging of the Brain and Heart and the International Conference on Functional Biomedical Imaging, 2007 - a summary of popular segmentation methods of medical images.

The object of the present invention is a method for anonymizing image data in medical images on which a face of a patient is visible. The anonymization is understood herein as a distortion of the image data (values of pixels) to such an extent as to prevent or at least make it more difficult to recognize the patient on the basis of the image, which is a volumetric reconstruction with a corresponding map of colors (or other methods - based on visual assessment, including any presentation of data or automatic image analysis). The anonymization cannot influence the diagnostic quality of the examination.

The anonymization may be directed to the whole facial skeleton and/or the patient's head or just a particular fragment, for example the face or a fragment of the patient's face. A selection of the region to be anonymized (or to be excluded from the anonymization) may be automatic, semiautomatic or manual. The characteristic points such as eyes, nose, lips, forehead line, chin line, ears (earlobes) etc may be automatically recognized. These characteristic points can be presented to the user, who can decide which of these characteristic points (and optionally a certain region around them) is to be anonymized (or to be excluded from the anonymization). The user can manually select (using a particular graphical interface, GUI) a region or regions to be anonymized.

The region to be anonymized is modified by a data encryption method, which may be reversible or irreversible. An encryption key can be derived from the user or can be generated automatically.

The output file or files with the modified image data are stored in a known standard, for example in a DICOM standard.

The medical images prepared in such a way may be presented using a standard software for processing the particular type of images, without the functionality of image de-anonymization. The resulting image will have a form of the examination with a distorted fragment or fragments of the image.

Medical images prepared in such a way may be also presented using a software having the image de-anonymization functionality - the resulting image will have a form of the original image (before the anonymization), if the user has the corresponding decryption key. If the user does not provide the decryption key or if the anonymization is irreversible, then the distorted image will be displayed.

The term "2D and 3D image analysis" as used herein relates to any method in the field of analysis and processing images which allows to determine, identify and separate a group of pixels representing characteristic anatomic regions. Image analysis is understood as determining at least one type of image features in a 2D and 3D space, from the following groups of features:
- features related to image histogram, statistical features, which can be obtained based on a co-occurrence matrix or a band matrix;
- features obtained on the basis of autocorrelation or autoregressive methods;
- features determined on the basis of a local image gradient, methods that analyze the inhomogeneities of a structure of a texture and analyze a local image entropy;
- features determined in a frequency domain by a Fourier transform, a continuous wavelet transform and their extensions (Contourlet, Surfacetet, Curvelet, Schearlet, Tetralet etc.), as well as wavelet statistical features (WSF);
- features determined on the basis of transforms such as the Hough transform and the Radon transform, on the basis of edge filters, gradient filters, a Gabor filter, nonlinear filters, logic filters, or tube detection filters (TDF);
- features related to a geometry of objects;
- features related to a shape of objects, a shape signature, the Freemam chains, the Fourier Description (FD) transformations, the Hausdorff distance;
- topological and morphological features, a density of edges, a fractal dimension, features determined on the basis of the Laplacian pyramid;
- features determined on the basis of filter banks;
- features determined by the use of Local Binary Patterns or Lewis filters and other methods for examination of irregularity of binary systems;
- as well as other features of an image texture and other features of the image.

Moreover, the image analysis is also understood as a 2D and 3D image segmentation, based on at least one of the following methods:
- a segmentation (binarization), with one or multiple thresholds, utilizing contours which base on nearest neighbor classifiers, clustering methods which use a boosting classifier or boosting trees;
- approaches based on region-growing and region-division methods, classification based on neural networks, an edge analysis;
- approaches based on correlation methods (correlation, autocorrelation coefficients) utilizing a finite-state automation, the hidden Markov models, the Markov chains, optimization methods such as evolution algorithms and other, statistical methods;
- a multi-scale and multi-resolution segmentation;
- approaches utilizing an SVM algorithm, a watershed algorithm together with its extensions, atlas-based image segmentation methods;
- a segmentation utilizing image registration methods and a previously generated model, shape matching methods;
- methods utilizing a gradient vector flow (GVF) etc;

The image analysis is understood herein as any method analyzing a geometry or shapes of objects (both in binary images and recorded in a gray-scale), in a space domain as well as in a frequency domain.

The image analysis is also understood herein as comprising multi-scale filtration methods such as the Hessain and other filters, methods based on parametric and nonparametric deformable models, graph models, methods based on a mathematical morphology, a discreet topology, and also labeling of objects included in the image.

The image analysis is also understood herein as comprising methods utilizing neural networks, statistical methods of classification, decision trees, methods based on pattern matching, optimization methods, modeling data with the use of the hidden Markov models (HMM), GMM models (Mixture of Gaussian Models), EM (Expectation-Maximization), clustering methods (also in a fuzzy version) and other methods classified to widely understood image (pattern) recognition issues, and also all other methods, which result in a description of the image in the numerical form.

Apart from the aforementioned, the term "analysis of the 2D and 3D images" also includes methods of features selection (sequential methods, analysis of a discrimination ability of a single feature, algorithms based on the evolution methods and others). It includes also noise reduction algorithms of a reference set, reduction (condensation) of the reference set or methods based on expert systems.

The term "analysis of the 2D and 3D images" also includes image processing operations such as a linear and nonlinear filtration of the image, algorithms based on a change or a correction of an image histogram, noise reduction algorithms, algorithms of contrast enhancement and correction, geometrical transformations, an interpolation, point operations and other.

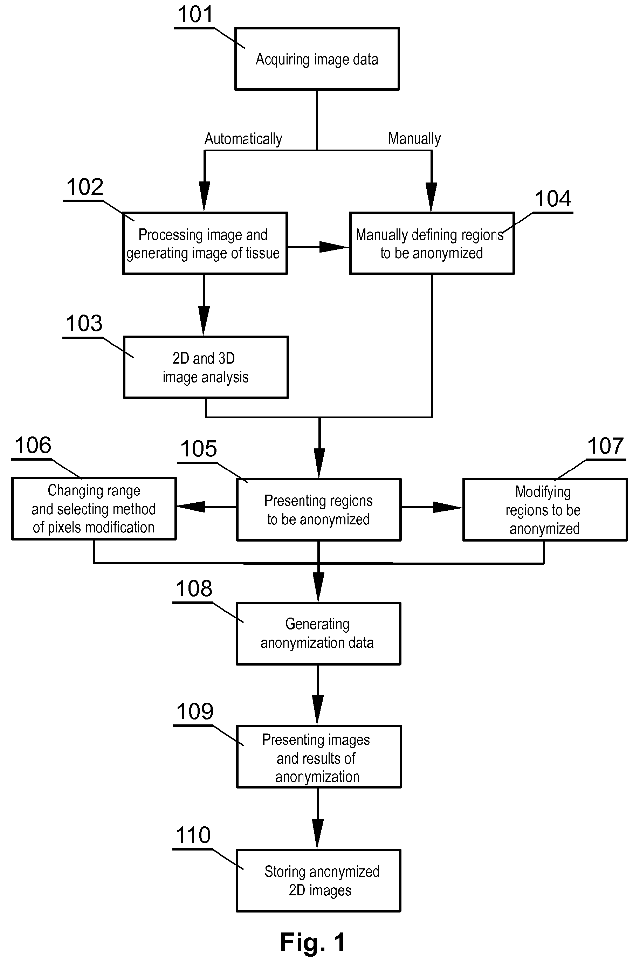

Fig. 1 shows a schematic of the method for anonymizing image data in the medical images.

In step 101 the image data is acquired from the medical image, such as the size of the image (in pixels), the origin of a coordinate system or an examination modality.

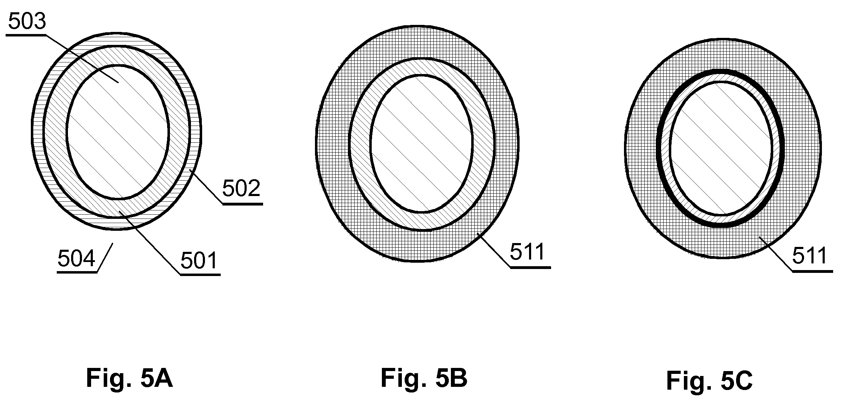

Next, if the data are to be processed automatically or semi-automatically, then in step 102 the image data is processed. The processing comprises the image segmentation, the purpose of which is the generation of a tissue image, for example as shown in Fig. 5A, which includes the separated regions of a bone tissue 501, a skin tissue 502, the rest of the tissue 503 and the background 504 (the air surrounding a patient's head).

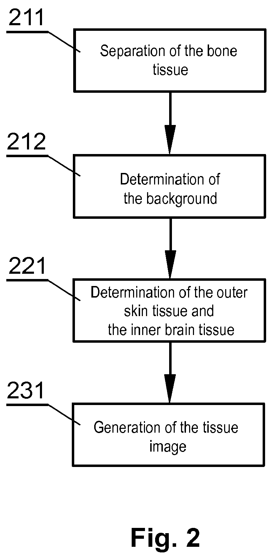

The segmentation may be a two-stage process as shown in Fig. 2.

The first stage of the segmentation 211 may include the separation of the bone tissue from the image data. For this purpose the maximization of entropy method may be used, described in the publication J. N. Kapur, P. K. Sahoo, and A. K. C. Wong, "A new method for gray-level picture thresholding using the entropy of the histogram," Computer Vision, Graphics, and Image Processing, vol. 29, no. 1, p. 140, 1985.

It allows to effectively determine a threshold that separates the bone tissue from other structures. The applied algorithm is based on the analysis of an image histogram. Conducted trials have shown that the threshold determined by this algorithm allows to properly separate the bone tissue form the other elements located on the image. A morphological closing of the object may be used in order to level the segmented bone tissue, delete small discontinuities and other disturbances (holes, insertions etc.). In step 212 the region of the background, comprising all of the pixels of the surroundings being beyond the patient's body, is determined.

The second stage of the segmentation may comprise the separation of classes associated with the soft tissue, and separation of the skin tissue (located on the surface of the patient's skull or body) from the brain tissue and from the remaining tissue located inside the patient's body.

In other words, this stage comprises the separation of the image data of the skin tissue, which covers the patient's head as well as the face, and the brain tissue, located inside the patient's skull. This stage of the segmentation may use the algorithm which analyzes 2D halftones (cross-sections) of the transverse plane, acquired in the first stage of classification.

In step 221 the regions of the outer skin tissue and the inner brain tissue (with respect to the bones of the skull) are determined. The methods based on components labeling, a discreet topology or morphological operators may be used. Preferably, a connected component algorithm described in the publication of H. Samet and M. Tamminen, "Efficient component labeling of images of arbitrary dimension represented by linear bintrees," IEEE Trans. Pattern Anal. Mach. Intell., vol. 10, no. 4, pp. 579-586, 1988 and the topological analysis algorithm may be used.

In step 231 the tissue image having the dimensions of the original image, comprising pixels having only one of four values, corresponding to four classes of the objects: the background (air) 504, the internal tissue (diagnostically relevant) 503, the external tissue - the skin tissue 502 (which can be anonymized) and the bone tissue 501 (which can be diagnostically relevant or not - depending on the user requirements) is generated.

In step 103 the analysis of the 2D and 3D image data is performed, for the purpose of an automatic detection of the regions to be anonymized. In this step the main anonymization region 511 is determined, which is schematically shown in Figs. 5B and 5C, and the characteristic regions of the face (eyes, a nose, lips etc.) are determined.

The main anonymization region 511 is the entire region determined for the particular examination as allowed for the anonymization. Its determination is based on the formation of the region around the skin tissue (extending to the background area) by the use of morphological operators. The region available for the anonymization is a sum of sets of pixels of the "skin tissue" class and the determined pixels of the region around the skin tissue. This region may contain (as shown in Fig. 5B) the segmented skin and the elements associated with it and the specified fragment of the space around the patient (background), and possibly additionally (as shown in Fig. 5C) also the particular part of the bone structure (by this manner, the deeper anonymization may be realized, which allows to remove the characteristic points of the bone structures - after their removal the reconstruction of the shape of the patient's face on the basis of the shape of the skull is impossible).

In order to prevent an expansion of the anonymization region to the bone tissue, the conditional morphological operators may be applied, for example a SKIZ operator (a dilation without images joining, a conditional erosion etc.), as described in the publication of R. Tadeusiewicz and P. Korohoda, "Komputerowa analiza i przetwarzanie obrazów", 2004 (Computer analysis and processing of images). The expansion of the main region of the anonymization by the SKIZ operator causes the growth of the region to be anonymized only in the direction of the background region, without entering on the bone tissue region.

The size of the anonymization region depends on the number of iterations of the SKIZ operator. It occupies the whole region of the skin tissue class and a part of the background region (which extends around the skin). If the user decides that the anonymization should cover also the part of the bone tissue, then before the execution of the aforementioned procedure, the region inside the bone tissue is determined, which is to be included into the main region of the anonymization. It is performed by the n-iterations of the conditional erosion or by the determination of the skeleton of the object and exclusion from the object, all of the pixels fulfilling the condition of a topological belongingness to its outer part (with respect to the skeleton) and an adequately distant (with respect to the specified metric) from this skeleton. Only the pixels of the skull, not the pixels of the structures belonging to the cervical spine, are subject to this procedure. Whether the particular pixel (or the object, namely a coherent in the topological sense set of pixels) is the object representing the skull, depends on the fulfillment of the condition of the belongingness to the bones of a skull class. This condition consists in the checking the geometrical features of the object.

Other procedures, like subtraction of images, may also be applied.

The detection of the characteristic features of the face may be conducted by the statistical classifier. It performs the analysis in three planes for each point (pseudo 3D analysis). A start point is determined by the analysis of the shape, performed in a transverse plane. The classifier for the analysis utilizes several methods describing the geometrical features and shape coefficients.

The classifier may utilize the FD (Fourier Descriptor) coefficients, as described in the publications:
- E. Persoon, P. Eric, and F. King-Sun, "Shape Discrimination Using Fourier Descriptors," IEEE Trans. Syst. Man Cybern., vol. 7, no. 3, pp. 170-179, 1977;
- Folkers and H. Samet, "Content-based image retrieval using Fourier descriptors on a logo database," in Object recognition supported by user interaction for service robots, 2002;
- Dengsheng Zhang, Z. Dengsheng, and L. Guojun, "Content-based shape retrieval using different shape descriptors: a comparative study," in IEEE International Conference on Multimedia and Expo, 2001. ICME 2001., 2001.

Moreover the classifier may also utilize the local image texture, as described in the publications:
- M. N. Do and M. Vetterli, "The contourlet transform: an efficient directional multiresolution image representation," IEEE Trans. Image Process., vol. 14, no. 12, pp. 2091-2106, Dec. 2005;
- L. da Cunha, J. Zhou, and M. N. Do, "The nonsubsampled contourlet transform: theory, design, and applications," IEEE Trans. Image Process., vol. 15, no. 10, pp. 3089-3101, Oct. 2006.

Each identified region is placed in the geometrical center of a parallelepiped with defined dimensions.

It is also possible, in step 104, to manually define the regions to be anonymized. This step may be performed by means of a module equipped with a graphical user interface (GUI), which allows the user to manually select the region or regions which are to be anonymized. The user may define the graphical points or lines of a polygon both in the 2D projection as well as by selecting the points on the volumetric reconstruction (3D). The list of the points or the straight lines drawn by the user forms a set of planes which describe the three-dimensional objects.

In other words, in the system according to the invention, the modification of sizes and a shape of the main anonymization region 511 and the determined characteristic region of the face are received; and/or the additional detected regions are received or the existing detected regions are deleted; and/or the range and the degree of the modification of the values of the image pixels is modified.

In step 105, the regions to be anonymized are presented, on the basis of the results from steps 103 and 104. Each of such regions constitutes a common part of the region selected by the user and the main anonymization region. It prevents the incidental anonymization of the contents relevant from the diagnostic point of view, which may be caused for example by: an inaccurate selection of the region, for the anonymization, by the user.

In step 106 the change of the range and the choice of the image pixels modification method (reversible or irreversible) may be performed, by using a module equipped with a graphical user interface (GUI), which allows to change and edit the regions to be anonymized. This module transfers parameters (for example in the form of variable/variables in the memory), which are received by the system according to the present invention and this module may enable the following functionalities:
- an addition, a deletion or a modification of the region to be anonymized;
- a choice, if the process of the anonymization has to include the bone tissue and if yes - to what extent;
- performing the "full" anonymization by choosing the whole main anonymization region;
- a choice between the reversible or irreversible anonymization;
- a choice of an encryption algorithm type and a definition of a password (for the reversible anonymization) or image distortion methods (addition of noise, random values of the pixels, geometrical distortions etc. - for the irreversible anonymization).

Moreover, in step 107 the modification of the images to be anonymized may be performed, by a module equipped with the graphical user interface (GUI).

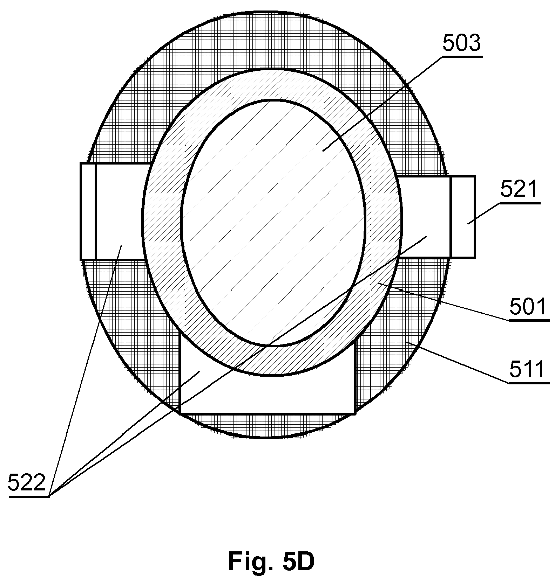

This step may be performed in two ways: the user may decide himself, which of the examination regions are to be anonymized (for example by determining the regions 521 shown in Fig. 5D), or to select from the list of the predefined regions, such as: surroundings of eyes, lips or a nose etc. For this purpose, the algorithm finding the characteristic points, in the particular examination image, such as: eyes, a nose, a forehead line, a chin line, ears, (earlobes) etc., may be used. Eventually, the region of the anonymization is an intersection (common part) of the selected region and the main anonymization region - these regions are referenced as 522 in Fig. 5D. The anonymization may be conducted on the region selected in this manner or on the whole main region excluding the selected region (for example, the user may want only the surroundings of eyes or lips to be visible).

The automatic recognition of the characteristic elements of the human face (such as eyes, a nose, lips etc.) is performed by the analysis of the pseudo 3D image. It is conducted in two stages. The first stage is the analysis of the binary object in the 2D projection (the transversal or front projection). This object is representing the human skull or the region of the skin tissue located on patient's face. The aim is to find the points suspected to being the characteristic points. When such a point is identified then the second stage of the analysis is performed. It is the pseudo 3D analysis. It relies on the determination of three sub-images, in three planes (transversal, frontal and sagittal) of the dimensions w x w, where w is the defined size, and the pixel selected in the first stage is the center of such sub-image. Next, the statistical analysis is performed with the use of the nearest neighbor classifier algorithm, and several features of the image (such as descriptors of the shape, the geometry or the image texture). In the result the particular pixel is qualified to the particular category (for example eyes, lips or a nose), or it is rejected. If the pixel is qualified as the characteristic point, then the characteristic region is created. It is the region around the characteristic point, of the dimensions set in the algorithm, for example the region around eyes has a form of a parallelepiped comprising both eyeballs and a certain region around.

In step 108, on the basis of the anonymization regions and the anonymization parameters defined in the previous steps, the anonymized data is generated. Preferably, the pixels of the medical image are subjected to the modification of position and/or the values of the pixels which enables the identification/recognition of the characteristic anatomical features of a patient while preserving the diagnostic value of the examination.

For this purpose, the list of the pixels to be anonymized is created.

Next, the minimal parallelepipeds are built around all the anonymization regions. Each anonymization region is processed separately.

In the case of the reversible anonymization, based on the created parallelepiped and the origin point of the coordinate system, a list of the pixels for the encryption is created. Such a list guarantees that the pixels will be arranged in the same sequence both in the process of encryption and later decryption. The user selects the type of the encryption algorithm and inputs the encryption key. After the encryption of a string of pixels a method is executed which is a function mapping the values obtained in the encryption process to the range of the values being out of the characteristic range for the particular type of the examination. This range is determined on the basis of the information about the modality of the examination. The encryption method is also a function which transforms the values of the encrypted pixels to the set of the values, being disjoint values with respect to the set of values which can be achieved for a particular type of the examination. Owing to this, it is possible to further unambiguously identify the pixels which had been anonymized.

In case of the irreversible anonymization, each pixel from the list of the anonymized pixels will be subjected to the process of their values distortion, preferably based on several different procedures, such as: addition of noise, random values of selected pixels, geometrical distortions etc.

Subsequently, the anonymized pixels are changed to the pixels obtained in the process of the anonymization.

In step 109 the images and the results of the anonymization are presented, by rendering them and displaying on a monitor of the anonymization results. Owing to this the user can observe on the display the effect of the performed anonymization. In this stage he can change their results, in a way to adjust it to its own needs. The user may be allowed to: add new anonymization regions, delete or modify the existing anonymization regions.

Next, in step 110 the anonymized images are stored, by storing the amended image data to the files compliant to a selected standard, for example the DICOM.

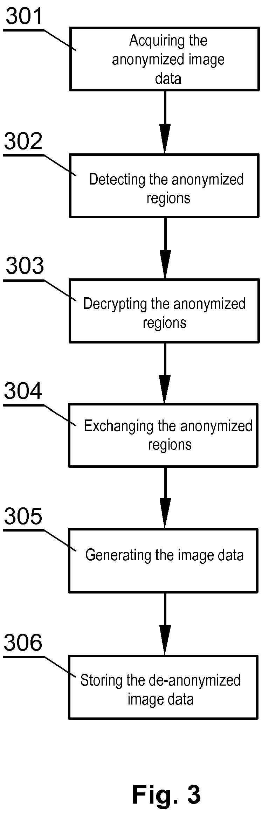

Fig. 3 shows the procedure of de-anonymization of anonymized data, by the reversible method.

In step 301 the anonymized image data is acquired from the medical image.

Next, in step 302 the anonymized regions are detected. The detection is based on the detection of all the pixels with the values exceeding the allowed value of the particular type of the examination. The minimal surrounding parallelepiped is created around each of the anonymization regions. On its basis and on the basis of the information about the origin of the coordinate system, the list of all the pixels is prepared while maintaining their appropriate sequence.

In step 303, using the decryption key provided by the user, the anonymized regions are decrypted. Each anonymized region is decrypted separately.

Subsequently, in step 304 the values of the distorted pixels are changed to the values obtained in the decryption process. Only the correct key guarantees the obtainment of the proper results of the de-anonymization.

In step 305, a stream of the image data to be presented to the user is generated.

In step 306 the de-anonymized image data may be stored at user's device in a selected standard, for example DICOM.

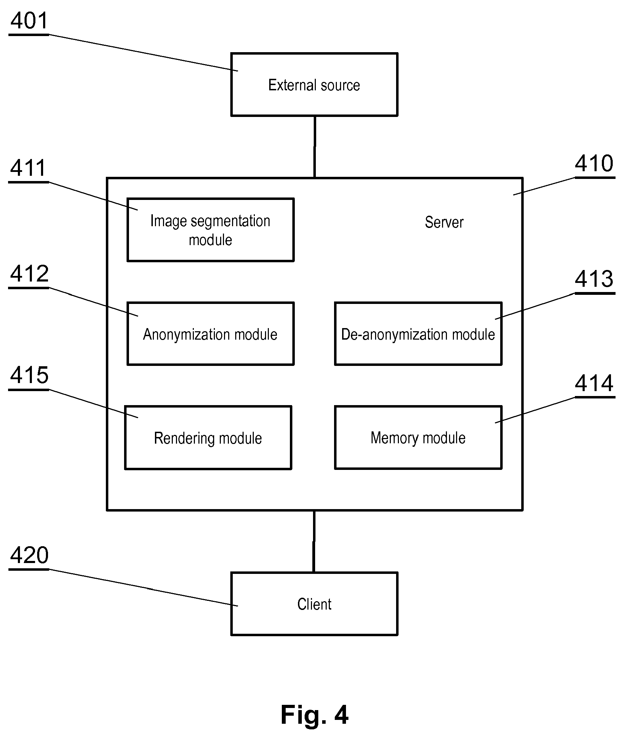

Fig. 4 show the schematic of a system for anonymizing image data from medical images. The system may be realized in a form of a server 410 comprising the following modules. An image segmentation module 411 is configured to perform steps 102-104 of the method presented in Fig. 1 on the basis of the image data acquired from the external source 401, for example it can be a CT or MRI imaging device or an external image repository.

An anonymization module 412 is configured to perform steps 106-108 of the method presented in Fig. 1. The resultant data of the anonymization module may be rendered in a module 415, configured to perform step 231 and stored in a memory 414 in order to realize step 109. A rendered image 415 may be shared via the server 410 with an external client 420, for example with a user terminal for browsing the anonymized data.

The client 420 may send to the server 410 data which are needed for the image de-anonymization, for example an individual client key for decryption of data or the anonymized images, which the client would like to view. The images are sent to the de-anonymization module, configured to perform steps 301-303 and cooperating with a rendering module 415, which in case of the de-anonymization procedure is configured to perform steps 304-305 of the procedure of Fig. 3, and the memory module 414 for storing the de-anonymized data.

The server 410 may be have a form of a dedicated electronic circuit (for example ASIC or FPGA) or a computer comprising a memory and being controlled by an adequate software. The server 410 may be embedded in the CT or MRI imaging device or may constitute a separate device.

The client's device 420 may be a typical desktop computer or a portable device, such as a smartphone or a tablet, equipped with an appropriate software. The client's software for cooperating with the server may have a form of a dedicated application, or the server may enable access to its functionality through an application executed on the server and available to the client via an Internet browser.

In another embodiment of the system, two separate servers may be used - a server for the anonymization comprising the image segmentation module, the anonymization module, the rendering module and the memory, and a server for the de-anonymization comprising the de-anonymization module, the rendering module and the memory.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A method for anonymizing image data in medical images, **characterized in that** it comprises the following steps:
- retrieving (101) the image data from the medical image;
- processing (102) the image data;
- analyzing (103) the 2D and 3D image data to automatically detect regions to be anonym ized;
- generating (108) anonymized data on the basis of the regions to be anonymized; and
- storing (110) the anonymized 2D images, by storing changed image data in a file compliant with a selected standard.

2. The method according to claim 1, **characterized by** further comprising retrieving (101) metadata characterizing the image data, such as an image size, an origin of a coordinate system or a modality of an examination.

3. The method according to claim 1, **characterized in that** processing the image data comprises segmenting the image to generate an image of tissues.

4. The method according to claim 3, **characterized by** performing the segmentation as a two-stage process, wherein the first stage of the segmentation (211) comprises separating a bone tissue from the image data and wherein the second stage of the segmentation comprises separating classes associated with a soft tissue and separating a skin tissue from a brain tissue and from the other tissues within the patient's body.

5. The method according to claim 1, **characterized in that** the automatic detection of the regions to be anonymized comprises determining a main anonymization region (511) and characteristic regions of a face.

6. The method according to claim 1, **characterized by** comprising, after analyzing (103) the 2D and 3D image data, the following steps:
- presenting (105) the regions to be anonymized, on the basis of the analysis of the 2D and 3D image data;
- receiving (107) a modification of sizes and shapes of the main anonymization region (511) and determined characteristic features of the face; and/or
- receiving additional regions or deleting existing detected regions; and/or
- modifying (106) a range and a degree of modification of values of pixels of the images.

7. The method according to claim 1, **characterized by** anonymizing the regions covering a head, a fragment of the head, a face or a fragment of the face.

8. The method according to claim 1, **characterized by** receiving (106) information on whether the anonymization process is to be reversible or irreversible.

9. The method according to claim 1, **characterized in that** generating (108) the anonymization data comprises creating a list of all pixels to be anonymized, and next creating minimal parallelepipeds around all the anonymization regions, wherein each anonymization region is processed separately.

10. The method according to claim 1, **characterized by** subjecting the pixels of the medical image to a modification of a position and/or a value, to prevent identification of characteristic anatomical features of a patient while preserving the diagnostic value of the examination.

11. The method according to claim 1, **characterized by** enabling the process of a de-anonymization, to allow recovering an initial state of the image data prior to the anonymization, wherein the data can be de-anonymized by subjecting it to the reversible anonymization based on data identifying the type of an encryption algorithm and an encryption key.

12. A computer program comprising program code means for performing all the steps of the computer-implemented method according to any of claims 1-11 when said program is run on a computer.

13. A computer readable medium storing computer-executable instructions performing all the steps of the computer-implemented method according to any of claims 1-11 when executed on a computer.

14. A system for anonymizing image data in medical images, **characterized in that** it comprises:
- a server (410) comprising an image segmentation module (411) configured to perform processing (102) the image data and analyzing (103) the 2D and 3D image data to automatically detect regions to be anonymized on the basis of the image data retrieved from an external source (401);
- an anonymization (412) module configured to perform generating (108) anonymized data on the basis of the regions to be anonymized and to share a rendered image (415) via the server (410) to an external client (420);
- wherein the client (420) is configured to send data, needed for a de-anonymization of the image, and the anonymized images to the server (410);
- a de-anonymization module configured to receive the data needed for the de-anonymization of the image and to receive the anonymized images and configured to de-anonymize in case when the image data was subjected to the reversible anonymization and when the module receives the correct data comprising the type of an encryption algorithm and an encryption key; and
- a memory module (414) for storing the de-anonymized images.
